# EUROPÄISCHE PATENTANMELDUNG

(11) **EP 1 843 158 A1**
(43) Veröffentlichungstag der Anmeldung: **10.10.2007**
(21) Anmeldenummer: 06007177.6
(22) Anmeldetag: 05.04.2006
(51) Int. Cl.: G01N 33/569

(54) **Nachweissystem für Krankheitserreger**

(71) Anmelder: Micronas Holding GmbH, 79108 Freiburg (DE)
(72) Erfinder: Klapproth, Holger, 79108 Freiburg (DE)
(74) Vertreter: Stürken, Joachim

(57) **Zusammenfassung**

Die vorliegende Erfindung betrifft allgemein das Gebiet der Biotechnologie. Insbesondere betrifft die Erfindung ein Nachweissystem zur Analyse bzw. Diagnostik von Krankheitserregern sowie zur Überwachung des Verlaufs einer Infektionskrankheit.

## Beschreibung

Die vorliegende Erfindung betrifft allgemein das Gebiet der Biotechnologie. Insbesondere betrifft die Erfindung ein Nachweissystem zur Analyse bzw. Diagnostik von Krankheitserregern sowie zur Überwachung des Verlaufs einer Infektionskrankheit.

Diagnostika und entsprechende Verfahren unter Verwendung derselben basieren zumeist auf der spezifischen Erkennung von Antikörpern im Blut infizierter Personen oder auf dem spezifischen Nachweis von Bestandteilen des Erregers wie z.B. Nukleinsäuren und Proteinen. Die meisten erregerspezifischen Nachweise erfolgen heute durch Anwendung der ELISA-Technik. Diese Technik setzt jedoch die Kenntnis und Verfügbarkeit eines geeigneten Epitops voraus, mit dem die gegebenenfalls in einer zu untersuchenden Probe infolge einer spezifischen Immunantwort vorhandenen Antikörper gebunden und nachgewiesen werden können. Sofern diese Voraussetzung nicht erfüllt ist, kann die Entwicklung entsprechender Nachweissysteme Monate oder sogar Jahre dauern.

Die vorliegende Erfindung basiert auf der Tatsache, dass sich viele Krankheitserreger wie insbesondere Mikroorganismen mittels PCR schnell analysieren und nachweisen lassen. Sofern ausreichend Informationen über die genetische Organisation des Erregers gewonnen werden können oder aus anderen Quellen vorliegen, lassen sich die für Polypeptide kodierenden Regionen des Genoms bestimmen und zur Bildung entsprechender Oligopeptide einsetzen. Diese Oligopeptide können anschließend mit Proben eines oder mehrerer zu untersuchenden Individuen inkubiert werden um festzustellen, ob und gegen welche Oligopeptide die Probe Antikörper aufweist. Da davon auszugehen ist, dass im Wege der Immunantwort nicht nur ein Antikörper sondern eine Mehrzahl von Antikörpern gebildet wird, lassen sich unter Anwendung kombinatorischer Chemie erregerspezifische Repertoires von Oligopeptiden erstellen, die für einen gegebenen Erreger spezifisch sind. Die verschiedenen Vertreter dieses spezifischen Oligopeptid-Repertoires können beispielsweise durch bekannte Verfahren zur Festphasensynthese hergestellt und zum Nachweis des Erregers durch Bindung mit Antikörpern verwendet werden.

Nutzt man diese Repertoires, um die Seren gesunder (nicht infiziert) und kranker (infiziert) Probanden miteinander zu vergleichen, lässt sich durch differenzielle Analyse der Bindungsereignisse sehr schnell erkennen, welche Oligopeptide des Repertoires von diagnostischem Wert sind. In der Regel können hierdurch mehrere Antikörper erfasst werden, die spezifisch an unterschiedliche Vertreter des Repertoires binden, wodurch die Zuverlässigkeit eines entsprechenden Nachweissystems deutlich gesteigert wird.

Da die Anzahl der von einem gegebenen Erreger nach Analyse seines Genoms möglichen Oligopeptide sehr hoch ist, wird nach einer bevorzugten Ausführungsform des erfindungsgemäßen Verfahrens zuerst geprüft, welche Oligopeptide zur Zusammenstellung des spezifischen Repertoires überhaupt geeignet sind. Diese Prüfung beinhaltet z.B. die EDV-gestützte Auswahl von Strukturen, die für eine effiziente Antikörperantwort zur Verfügung stehen, wobei insbesondere die Zugänglichkeit der Strukturen an der Erregeroberfläche eine wichtige Rolle spielt. Als Ergebnis dieser Untersuchungen wird eine hinsichtlich des gewünschten Bindungsverhaltens optimierte Teilmenge der insgesamt möglichen Oligopeptide erhalten, welche anschließend zur Etablierung des oben genannten erregerspezifischen Repertoires unter Verwendung verschiedener Seren gesunder und kranker Individuen untersucht wird.

Besonders geeignete Bereiche von Oligo- oder Polypetidketten zeichnen sich beispielsweise aus durch einen mindestens 30%igen Gehalt an den immunogenen Aminosäuren K, R, E, D, Q, N sowie durch einen signifikanten Anteil von geladenen oder hydrophilen Gruppen. Die Sequenzen werden zusätzlich gegen die Proteinesequenzen des Wirtsorganismus abgeglichen, da Sequenzen, die auch im Wirtsorganismus vorkommen, i.d.R. nicht immunogen sind oder bei einer Immunantwort zu unerwünschten Wirkungen führen. Die Mindestkettenlänge der Oligonukleotide liegt in der Regel bei 5 Aminosäuren, wobei längere Ketten bevorzugt sind.

Bei einer erfolgten Bindung von Antikörpern kann durch die Markierung der Antikörper mittels sekundärer Antikörper (z.B. Anti-human IgG) sehr schnell erkannt werden, welche Peptide das Repertoire umfassen sollte. Durch die Messung anderer Immunglobulin-Subklassen (z.B. IgM, IgA) kann unter Verwendung desselben Repertoires auch eine Aussage zur aktuellen Phase des Verlaufs einer Krankheit oder Infektion getroffen werden, da z.B. IgM-Immunantworten nach erfolgter Infektion sehr schnell auftreten und von späteren IgG-Antworten abgegrenzt werden können. Durch den Vergleich mit Seren gesunder Probanden ohne Antikörper gegen den Erreger lässt sich die Sicherheit des erfindungsgemäßen Nachweissystems deutlich erhöhen.

Sowohl für die obigen Schritte zur Etablierung eines erregerspezifischen Repertoires als auch für die Herstellung erfindungsgemäß geeigneter diagnostischer Vorrichtungen werden die einzelnen Vertreter der obigen optimierten Teilmenge bzw. des Oligopeptid-Repertoires nach einer bevorzugten Ausführungsform unter Anwendung bekannter Festphasenverfahren synthetisiert, wobei die *in situ* Synthese auf einem Biosensor oder Biochip besonders bevorzugt ist.

Erfindungsgemäß sind zur Herstellung diagnostischer Vorrichtungen sämtliche Vorrichtungen des Standes der Technik geeignet, die den diagnostischen Nachweis eines Erregers in einer zu untersuchenden Probe über die spezifische Wechselwirkung zwischen Antikörper und Ligand (Oligopeptid) gewährleisten.

Die erfindungsgemäßen diagnostischen Verfahren und Vorrichtungen ermöglichen die gewünschtenfalls gleichzeitige Klärung verschiedener Fragestellungen. Erstens lässt sich feststellen, ob ein zu untersuchendes Individuum mit dem gesuchten Krankeitserreger infiziert ist. Zweitens kann durch Vergleich der für verschiedene Immunglobulin-Subklassen gemessenen Werte (IgM vs. IgG) die Phase des Verlaufs einer infektionsbedingten Erkrankung ermittelt werden. Drittens kann durch quantitative Erfassung der gebundenen Antikörper festgestellt werden, wie effizient die Immunantwort des infizierten Individuums ist.

Ferner ist es durch Vergleich von Individuen mit festgestellter effizienter Immunantwort mit Individuen mit festgestellter ineffizienter Immunantwort erfindungsgemäß möglich festzustellen, gegen welche Epitope des Erregers neutralisierende Antikörper gebildet werden. In Kenntnis der auf diese Weise erhaltenen Befunde lassen sich dann auf üblichem Wege Impfstoffe entwickeln bzw. spezifische Antikörper herstellen, die zur Therapie bzw. Therapieunterstützung eingesetzt werden können.

Die Erfindung wird nachfolgend anhand eines nicht beschränkenden Beispiels näher erläutert.

### Beispiel

### Nachfolgend wird der Nachweis neutralisierender Antiköper für das HA-Gen von Influenzaviren beschrieben.

Die Nukleinsäuresequenz des HA-Gens (Haemagglutinin) der meisten Influenzaviren ist bekannt. Die verschieden Subtypen unterscheiden sich nur geringfügig, so dass die Nukleinsäuresequenz selbst für neue Varianten schnell mittels z.B. Consensus-PCR und anschließender Sequenzierung erhalten werden kann. Anhand dieser Sequenzdaten kann dann sehr schnell die Aminosäureabfolge des Proteins bestimmt werden. Die Nukleinsäuresequenz weist ca. 1778 Nukleotide auf. Daraus kann ein Protein von maximal 592 Aminosäuren hergestellt werden. Die genaue Anzahl der Aminosäuren (AS) kann mittels geeigneter DNA-Analysesoftware (z.B. DNASTAR, DNASTAR Inc., Madison, WI) errechnet werden. Anhand der ermittelten AS-Sequenz werden dann für die Analyse auf einem Chip unterschiedliche Oligopeptide mit einer jeweiligen Kettenlänge von z.B. 20 Aminosäuren synthetisiert. Entsprechende Verfahren zur parallelen Synthese sind dem Fachmann geläufig. Im Rahmen dieser Synthese werden überlappende Oligomere hergestellt, die jeweils um 5 Aminosäuren in der Sequenz verschoben sind. Dabei entstehen ca. 115 Fragmente mit der vorgesehenen Länge. Auf diese Fragmente wird dann Serum von erkrankten Personen gegeben, und nach Inkubation (1h, RT, Serum 1:10 mit PBS-T BSA verdünnt) wird mit Waschpuffer gewaschen und die Bindung der Antikörper des Probanden gegen das Virus mit einem HRPmarkierten Sekundärantikörper nachgewiesen. Dabei wird speziell darauf geachtet, dass gesunde Personen als Referenz gemessen werden, um unspezifische Bindungsereignisse auszuschließen. Sollten diese Referenzdaten bereits vorliegen, kann der Abgleich unter Verwendung dieser Datensätze erfolgen. Als Sekundärantikörper kann in diesem Fall ein Anti-human-IgG Antikörper verwendet werden. Soll eine frische Immunantwort nachgewiesen werden, wird statt eines Anti-IgG ein Anti-IgM Antikörper verwendet. Die Bindung dieser Antikörper zeigt, gegen welche Epitope des Virusproteins eine Immunantwort stattgefunden hat. Will man nun zusätzlich die Effizienz der Immunantwort testen, so werden die gebundenen Antikörper des Probanden nach der Bindung mit einem milde denaturierenden (oder einem chaotropen) Agenz wie z.B. Harnstoff oder Guanidiniumisothiocynanat (GITC) behandelt, um Antikörper, die mit geringer Bindungsenergie binden (= geringe Affinität), wieder zu entfernen. So werden nur hochaffine Antikörper nachgewiesen und dadurch eine effiziente Immunantwort aufgezeigt.

## Patentansprüche

1. Immunologisches Nachweissystem für Krankheitserreger, umfassend eine diagnostische Vorrichtung mit einem erregerspezifischen Repertoire von Oligopeptiden, deren Sequenz aus dem Genom des Erregers abgeleitet worden ist.

2. Immunologisches Nachweissystem nach Anspruch 1, **dadurch gekennzeichnet, dass** das Repertoire nur Oligopeptide umfasst, die mit antigenen Strukturen des Erregers korrespondieren.

3. Immunologisches Nachweissystem nach Anspruch 2, **dadurch gekennzeichnet, dass** die antigenen Strukturen an der Zelloberfläche des Erregers exprimiert werden und in der Lage sind, in einem Individuum eine Immunantwort auszulösen.

4. Immunologisches Verfahren zum Nachweis des Vorhandenseins eines Erregers in einem zu untersuchenden Individuum, umfassend die folgenden Schritte:
(a) Inkubation eines Repertoires von Oligopeptiden, deren Sequenz aus dem Genom des Erregers abgeleitet worden ist, mit Serum des zu untersuchenden Individuums, unter geeigneten Bedingungen zur Ausbildung von Oligopeptid/Antikörper-Komplexen; und
(b) Auswertung des Schrittes (a) durch Detektion entstandener Komplexe;
wobei das Vorhandensein des Erregers in dem zu untersuchenden Individuum durch Detektion mindestens eines in Schritt (b) entstandenen Komplexes nachgewiesen wird.

5. Verfahren nach Anspruch 4, **dadurch gekennzeichnet, dass** das in Schritt (a) eingesetzte Repertoire nur Oligopeptide umfasst, die mit antigenen Strukturen des Erregers korrespondieren.

6. Verfahren nach Anspruch 5,**dadurch gekennzeichnet, dass** die antigenen Strukturen unter solchen Strukturen ausgewählt sind, die an der Zelloberfläche des Erregers exprimiert werden und in der Lage sind, in einem Individuum eine Immunantwort auszulösen.

7. Verfahren nach einem der Ansprüche 4 bis 6, **dadurch gekennzeichnet, dass** der Nachweis in der Weise durchgeführt wird, dass zwischen gebundenen Antikörpern verschiedener IgG-Klassen unterschieden werden kann.

8. Verfahren nach Anspruch 7, **dadurch gekennzeichnet, dass** die verschiedenen IgG-Klassen aus IgG, IgM und IgA ausgewählt werden.

9. Verfahren nach einem der Ansprüche 4 bis 8, ferner umfassend den Vergleich der für verschiedene Immunglobulin-Subklassen gemessenen Werte zur Erfassung der aktuellen Phase oder des Verlaufs einer durch den Erreger in dem Individuum ausgelösten Erkrankung.

10. Verfahren nach Anspruch 9, **dadurch gekennzeichnet, dass** für den Vergleich die für die Immunglobulin-Subklassen IgM und IgG gemessenen Werte herangezogen werden.
